# EUROPÄISCHE PATENTSCHRIFT

(11) **EP 0 431 487 B1**
(45) Veröffentlichungstag und Bekanntmachung des Hinweises auf die Patenterteilung: **26.10.1994**
(21) Anmeldenummer: 90122931.0
(22) Anmeldetag: 30.11.1990
(51) Int. Cl.: C07C 43/29, C07C 43/16

(54) **Verfahren zur Herstellung von 3,4'-Dichlor-diphenylether**
Process for preparing 3,4'-dichlorodiphenyl-ether
Procédé de préparation de 3,4'-dichloro-diphényl-éther

(30) Priorität: 05.12.1989 DE 3940130
(43) Veröffentlichungstag der Anmeldung: 12.06.1991
(73) Patentinhaber: HOECHST AKTIENGESELLSCHAFT, 65926 Frankfurt am Main (DE)
(72) Erfinder: Papenfuhs, Theodor, Dr., W-6000 Frankfurt am Main (DE); Schubert, Hans, Dr., W-6233 Kelkheim (Taunus) (DE); Hess, Reiner, Dr., W-6200 Wiesbaden (DE)

(56) Entgegenhaltungen:
- US-A- 4 766 253
- CHEMICAL ABSTRACTS, Band 109, Nr. 9, 29. August 1988, Seite 658, Zusammenfassung Nr. 73126z, Columbus, Ohio, US; H. OSHINO et al, "Process for preparation of 3,4'-dichlorodiphenyl ether as an intermediate for agrochemicals, pharmaceuticals, and polymers"; & JP-A-62 281 837
- CHEMICAL ABSTRACTS, Band 110, Nr. 17, 24. April 1989, Seite 684, Zusammenfassung Nr. 153894j, Columbus, Ohio, US; H. OSHINO et al, "Process for the preparation of 3,4'-dichlorodiphenyl ether as intermediate for 3,4'-diaminodiphenyl ether"; & JP-A-63 41 434

## Beschreibung

3,4'-Dichlor-diphenylether ist ein wichtiges aromatisches Vorprodukt und dient u.a. zur Herstellung von 3,4'-Diamino-diphenylether, einem wichtigen Rohstoff zur Synthese von Hochmodul-Aramiden, dient aber auch als Grundkörper zur Herstellung von Pflanzenschutzmitteln.

Die Chlorierung von Diphenylether, die durch Reaktion von Diphenylether mit SO₂Cl₂ (NL 75/4110 v. 18.10.76) in Gegenwart schwefelhaltiger Katalysatoren durchgeführt wird, führt nicht zu dem gewünschten Produkt, Sondern zu 2,4'-Dichlor-diphenylether neben 4,4'-Dichlor-diphenylether gemäß dem Reaktionsschema 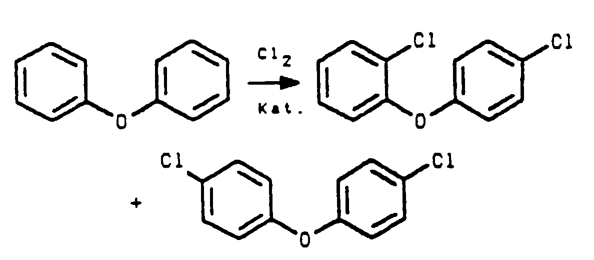
Durch geschickte Wahl des Katalysators gelingt es zwar, das o-/ p-Verhältnis bei der Zweitchlorierung zu beeinflussen (BE 827 912, US 39 20 757, US 37 93 377; alle Dow Chemical Co.). Es gelingt aber auf diese Weise nicht, d.h. durch direkte Chlorierung, den gewünschten 3,4'-Dichlor-diphenylether herzustellen.

Synthetisiert man das gewünschte Produkt durch Diphenylether-Synthese nach Ullmann (Houben-Weyl, Bd. VI/3, Seite 86), so gibt es folgende Alternative für die Auswahl der Ausgangsprodukte: 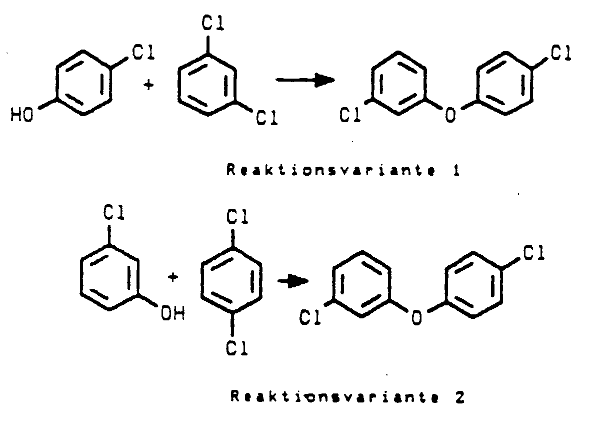
1. Umsetzung von 3-Chlor-phenol mit 1,4-Dichlorbenzol
2. Umsetzung von 4-Chlor-phenol mit 1,3-Dichlorbenzol
Zur technischen Herstellung würde aus Gründen der Verfügbarkeit der Ausgangsprodukte und der leichteren destillativen Trennung nicht umgesetzter Reste der Ausgangsverbindungen vom Produkt die erste Variante bevorzugt.

So setzen Oshino et al. (JP 62/281837 vom 7.12.1987) Natrium-4-phenolat, 1,3-Dichlor-benzol und wäßrige Natronlauge um, destillieren Wasser ab und rühren in Lösung (Dimethylformamid als Lösungsmittel) unter Zugabe von Kupfer(II)-acetat-dihydrat bei 155-165 °C über 18 Stunden. Über die Aufarbeitung wird nichts gesagt: es werden hier 88,8 % Ausbeute erhalten. Auch die stöchiometrischen Verhältnisse werden nicht geklärt.

Nachteilig an diesem Verfahren sind zum einen seine lange Umsetzungsdauer, zum anderen die Verwendung des Lösemittels Dimethylformamid, das sich als thermisch nicht sehr stabil erwies und deshalb weitgehend aus Gründen der Bildung von Zersetzungsprodukten aus technischen Prozessen eliminiert wurde.

In einer anderen Literaturstelle setzt Rauber (US 47 66 253 vom 23.8.1988) R₋C₆H₄₋OX (X= 1 Äquivalent Alkali oder Erdalkali) mit einem Überschuß von 3-15 Mol 1,3₋Cl₂₋C₆H₄ in Gegenwart eines Kupferkatalysators (hier CuO) mehrere Stunden bei 120-220 °C (im Beispiel 150 °C) um, wobei 0,003-3 Mol eines aprotischen Lösemittels zugegen sein müssen (im Beispiel N,N-Dimethylacetamid). Es werden hierbei nach einer nicht näher skizzierten Aufarbeitung 80 % Ausbeute an 3,4'-Dichlor-diphenylether erzielt.

Neben der nicht näher skizzierten Aufarbeitung und der mäßigen Ausbeute bietet diese Variante den Nachteil des Einsatzes eines Alkali- oder Erdalkali-phenolates, das in einer vorgeschalteten Synthese zunächst hergestellt werden muß. Mit dieser zweistufigen Reaktionsweise ist - bei bescheidener Ausbeute- eine technische Herstellung des 3,4'-Dichlor-diphenylethers nur wenig ökonomisch durchzuführen.

Oshino et al. berichten in einer anderen Veröffentlichung (JP 63/41434 vom 22.2.1988) über eine andere Verfahrensweise zur Herstellung von 3,4'-Dichlor-diphenylether:
Natrium-4-chlor-phenolat wird mit 1,3-Dichlor-benzol in flüssigen aliphatischen Glykolethern in Gegenwart von Cu-Katalysatoren zum gewünschten 3,4'-Dichlor-diphenylether umgesetzt. Im Beispiel werden
4-Chlor-phenol und 1,3-Dichlor-benzol mit wäßriger Natronlauge zum Natrium-4-chlor-phenolat umgesetzt, wobei das gebildete Reaktionswasser und das mit der Natronlauge eingetragene Wasser abdestilliert werden (kein Azeotrop).

Im Folgeschritt wird der Rückstand (es ist also nahezu alles Wasser abdestilliert worden) mit Diethylenglykoldiethylether aufgenommen und in Anwesenheit von Cu(I)Cl bei 155-160 °C 18 Stunden lang umgesetzt, um eine Ausbeute von 87,2 % zu erhalten, wobei nach dem Beschreibungstext nicht feststeht, ob es sich um isolierte oder chromatographisch bestimmte Ausbeute handelt.

Nachteilig an dieser Verfahrensweise ist neben der langen Reaktionszeit der Einsatz von Diglykol-dialkylethern, die über die ganze Palette hinweg einen relativ niedrigen Zündpunkt besitzen (unter 200 °C) und deshalb bei technischer Herstellung einen erhöhten Sicherheitsaufwand erfordern (Ausstattung der Herstellungsanlage nach ExT4), der die Anlage verteuert, bzw. in einer vorhandenen Anlage Zusatzkosten verursacht. Auch nach diesem geschilderten Verfahren ist also die ökonomisch günstige Herstellung von 3,4'-Dichlor-diphenylether nicht möglich.

Überraschenderweise ist es dennoch gelungen, auf der Basis von 4-Chlor-phenol und 1,3-Dichlor-benzol, also gängigen, technisch verfügbaren Ausgangsprodukten, in Gegenwart von Kaliumcarbonat als Hilfsbase, die das Ausschleppen großer Wassermengen - verglichen mit dem Eintrag wäßriger Natronlauge - erspart und einem geeigneten "Lösungsvermittler" in Anwesenheit einer Kupferverbindung als Katalysator, durch geeignete Verfahrensweise zu einem ökonomisch und ökologisch günstigen Herstellungsverfahren für 3,4'-Dichlor-diphenylether zu gelangen.

Gegenstand der Erfindung ist somit ein verbessertes Verfahren zur Herstellung von 3,4'-Dichlor-diphenylether aus 1,3-Dichlorbenzol und 4-Chlor-phenol, dadurch gekennzeichnet, daß man 1 Mol 4-Chlor-phenol mit 2 bis 6 Mol, vorzugsweise 4 bis 5 Mol, vorgelegtem 1,3-Dichlorbenzol und 1 bis 3 Mol, vorzugsweise 1 bis 1,5 Mol, Kaliumcarbonat in 1 bis 5 Mol, Vorzugsweise 1 bis 2 Mol, eines dipolar-aprotischen Lösungsvermittlers, der oberhalb 160 °C siedet, wie beispielsweise Dimethylacetamid, Sulfolan, Dimethylsulfoxid, vorzugsweise N-Methyl-pyrrolidon, bei Temperaturen von 160 bis 190 °C, vorzugsweise 170 bis 180 °C unter Rühren mischt, anschließend 0,01 bis 1 Mol-%, vorzugsweise 0,1 bis 0,9 Mol-% basisches Kupfercarbonat der Formel Cu(OH)₂·CuCO₃·0,5 H₂O zugibt, dann unter Rühren auf 170 bis 173 °C unter Abdestillieren des gebildeten Wassers erhitzt, anschließend gegebenenfalls noch 4-Chlor-phenol zusetzt mit der Maßgabe, daß von Anfang an die anwesende Menge 1,3-Dichlorbenzol mengenmäßig stets um 1 bis 3 Mol größer ist als die insgesamt zugegebene Menge 4-Chlor-phenol, und gegebenenfalls noch basisches Kupfercarbonat der genannten Formel zugibt, falls anfangs weniger als 0,1 Mol-% zugesetzt worden war, innerhalb des letztgenannten Temperaturbereichs (etwa 170 bis etwa 173 °C) weiterrührt und schließlich nach Abkühlenlassen aufarbeitet.

Die Reaktionsdauer beträgt nach beendeter Wasserauskreisung etwa 2-6 Stunden und bei Nachdosierung von 4-Chlor-phenol zusätzlich nochmals etwa 2-6 Stunden, also maximal etwa 4-10 Stunden.

Das Aufarbeiten nach dem Abkühlenlassen des Reaktionsgemisches wird so vorgenommen, daß man nach Abtrennen des angefallenen Rückstandes des eingesetzten basischen Kupfercarbonats und der gebildeten Salze, die noch mit 1,3-Dichlorbenzol gewaschen werden, auf den pH-Wert 6 bis 6,5 stellt und dann destillativ aufarbeitet.

Das Verfahren wird zweckmäßigerweise bei Normaldruck durchgeführt. Es kann aber auch bei Überdruck gearbeitet werden, sofern in Gegenwart eines dipolar-aprotischen Lösungsvermittlers, der unterhalb 160 °C siedet, wie beispielsweise Dimethylformamid, gearbeitet wird.

Ferner kann das Verfahren sowohl diskontinuierlich als auch kontinuierlich durchgeführt werden.

Bei der Aufarbeitung werden zunächst die "Leichtsieder" (NMP, 1,3-Dichlor-benzol und 4-Chlor-phenol) über eine kurze Kolonne (3-5 Böden) abgetrennt. Dann wird der gewünschte 3,4'-Dichlor-diphenylether ohne Kolonne einfach überdestilliert, wobei eine destillierte Ausbeute von über 90 % in sehr hoher Reinheit (GC > 99 %) erhalten wird.

Beim erfindungsgemäßen Verfahren werden drei Vorteile erkenntlich:
1. Die Raumzeit-Ausbeute liegt wegen der kurzen Umsetzungsdauer und der doppelten Zudosierung von 4-Chlor-phenol deutlich über derjenigen der oben geschilderten bekannten Verfahren. Man hat den zur Reaktion erforderlichen Überschuß von 1,3-Dichlor-benzol gegen 4-Chlor-phenol gewährleistet, ohne bei der Raumausbeute große Einbußen hinnehmen zu müssen.
2. Durch die geschilderte destillative Aufarbeitung ist es möglich, den abdestillierten "Leichtsiederanteil" wieder in die Folgereaktionen zurückzuführen. Auf diese Weise kann das "Hilfslösemittel" N-Methylpyrrolidon ohne weitere Ergänzung im Kreis gefahren werden.
3. Durch die Verwendung von N-Methyl-pyrrolidon als dipolar-aprotischem "Lösungsvermittler" hat man ein temperaturstabiles System mit hoher Zündtemperatur und geringem toxischen Potential, dessen technische Verfügbarkeit gewährleistet ist.

Insgesamt ist das erfindungsgemäße Verfahren durch Verwendung eines geeigneten "Lösungsvermittlers", eines geeigneten Kupferkatalysators und der Wahl der Verfahrensweise der doppelten Dosierung der Bezugskomponente 4-Chlor-phenol ein ökonomisch und ökologisch (durch Rückführung aller erneut einsetzbaren Komponenten) sehr günstiges Verfahren mit hoher Ausbeute an einem hochreinen 3,4'-Dichlor-diphenylether.

Das nachstehende Beispiel soll insbesondere die technische Durchführung des erfindungsgemäßen Verfahrens illustrieren, ohne letzteres darauf zu beschränken.

### Beispiel 1

In einem 2,5-m³-V₄A-Apparat mit Rührer und Gaseinleitung werden nach zweimaligem Evakuieren und Belüften mit Stickstoff 1740 kg 1,3-Dichlorbenzol (10 kmol), 260 kg 4-Chlor-phenol (2,023 kMol) und 600 kg N-Methylpyrrolidon (231 Gew.-%, bezogen auf Einsatz 4-Chlor-phenol) eingetragen. Unter schwachem Stickstoffstrom werden nun noch 310 kg Kaliumcarbonat, gekörnt (2,246 kMol) und 3 kg (0,013 kmol) basisches Kupfercarbonat zugesetzt. Der Behälterinhalt wird unter Rühren auf 170-173 °C erhitzt, wobei Wasser aus der Reaktion des Phenols mit Kaliumcarbonat und der Zersetzung des basischen Kupfercarbonats über einen Spritzschutz abdestilliert. Das Reaktionsgemisch wird 3 Stunden bei dieser Temperatur unter Rühren gehalten, bevor zunächst nochmals 3 kg basisches Kupfercarbonat zugegeben werden und später nochmals 260 kg (2,023 kMol) 4-Chlor-phenol langsam flüssig eindosiert werden. Der Ansatz wird nochmals 5 Stunden lang unter Rühren auf 170-175 °C gehalten, bevor auf 20-25 °C kaltgerührt wird.

Die festen Anteile des Reaktionsgemisches (gebildetes Kaliumchlorid und Kupferoxid aus dem Katalysator) werden über eine Drucknutsche abgetrennt und mit 300 kg 1,3-Dichlor-benzol produktfrei gewaschen. Das Filtrat wird zunächst durch Zugabe wäßriger 30 %iger Salzsäure auf pH 6-6,5 eingestellt, dann über Spritzschutz das Wasser bis 170 °C Übergangstemperatur abdestilliert und anschließend in eine 3 m³-V₄A-Blase zur Fraktionierung über eine Kolonne mit Sulzer-Packung (25-27 theoretische Böden) eingetragen.

Dort werden im Vakuum von zunächst 20 mbar die leichtsiedenden Bestandteile des Reaktionsgemisches bis zu einer Sumpftemperatur von 150 °C mit einem Rücklaufverhältnis von 1:3 abdestilliert, wobei das gesamte 1,3-Dichlorbenzol und N-Methyl-pyrrolidon sowie nicht umgesetzes 4-Chlor-phenol übergehen, die nach GC-Kontrolle der Zusammensetzung des Destillats in den Folgeansatz zurückgeführt werden. Der Sumpf dieser Fraktionierung wird direkt -ohne Kolonne - über Spritzschutz aus dem V₄A-Apparat abdestilliert. Man erhält hier im stationären Zustand eine Ausbeute von 880 kg (entsprechend 91 % der Theorie, bezogen auf den Einsatz an 4-Chlor-phenol) an 3,4'-Dichlor-diphenylether, der nach GC eine Reinheit von > 99 % besitzt.

### Beispiel 2 (Vergleichsbeispiel)

In einem 4-1-Vierhalskolben mit Rührer, Innenthermometer und beheiztem Tropftrichter, sowie einer Feststoffzugabe werden 1740 g (10 Mol) 1,3-Dichlor-benzol, 257 g 4-Chlor-phenol und 310 g Kaliumcarbonat nach Spülen mit Stickstoff vorgelegt. Das Gemisch wird unter Rühren auf 170-175 °C erhitzt und 10 Stunden lang bei dieser Temperatur gehalten, bevor gaschromatographisch der Umsatz gemessen wird. Man erhält einen Umsatz der Komponenten zum gewünschten 3,4'-Dichlor-diphenylether von unter 5 % (Fl. % der Auswertung).

## Patentansprüche

1. Verfahren zur Herstellung von 3,4'-Dichlor-diphenylether aus 1,3-Dichlorbenzol und 4-Chlor-phenol, dadurch gekennzeichnet, daß man 1 Mol 4-Chlor-phenol mit 2 bis 6 Mol vorgelegtem 1,3-Dichlorbenzol und 1 bis 3 Mol Kaliumcarbonat in 1 bis 5 Mol eines dipolar-aprotischen Lösungsvermittlers, der oberhalb 160 °C siedet, bei Temperaturen von 160 °C bis 190 °C unter Rühren vermischt, anschließend 0,01 bis 1 Mol-% basisches Kupfercarbonat der Formel Cu(OH)₂·CuCO₃·0,5 H₂O zugibt und unter Rühren auf 170 bis 173 °C unter Abdestillieren des gebildeten Wassers erhitzt, anschließend gegebenenfalls noch 4-Chlor-phenol zusetzt mit der Maßgabe, daß von Anfang an die anwesende Menge 1,3-Dichlorbenzol molmäßig stets um 1 bis 3 Mol größer ist als die insgesamt zugegebene Menge 4-Chlor-phenol, und gegebenenfalls noch basisches Kupfercarbonat der genannten Formel zugibt, falls anfangs weniger als 0,1 Mol-% zugesetzt worden war, innerhalb des letztgenannten Temperaturbereichs weiterrührt und schließlich nach Abkühlenlassen aufarbeitet.

2. Verfahren nach Anspruch 1, dadurch gekennzeichnet, daß man in Gegenwart von 0,1 bis 0,9 Mol-% basischem Kupfercarbonat umsetzt.

3. Verfahren nach mindestens einem der Ansprüche 1 und 2, dadurch gekennzeichnet, daß man in Gegenwart von 1 bis 2 Mol eines dipolar-aprotischen Lösungsvermittlers, der oberhalb 160 °C siedet, umsetzt.

4. Verfahren nach mindestens einem der Ansprüche 1 bis 3, dadurch gekennzeichnet, daß man in N-Methylpyrrolidon als Lösungvermittler umsetzt.

5. Verfahren nach mindestens einem der Ansprüche 1 bis 4, dadurch gekennzeichnet, daß man bei Temperaturen von 170 bis 180 °C umsetzt.

6. Verfahren nach mindestens einem der Ansprüche 1 bis 5, dadurch gekennzeichnet, daß man mit 1 bis 1,5 Mol Kaliumcarbonat umsetzt.

7. Verfahren nach mindestens einem der Ansprüche 1 bis 6, dadurch gekennzeichnet, daß man bei Normaldruck umsetzt.

8. Verfahren nach mindestens einem der Ansprüche 1 bis 6, dadurch gekennzeichnet, daß man bei Überdruck in Gegenwart eines dipolar-aprotischen Lösungsvermittlers, der unterhalb 160 °C siedet, umsetzt.

9. Verfahren nach mindestens einem der Ansprüche 1 bis 8, dadurch gekennzeichnet, daß man diskontinuierlich oder kontinuierlich arbeitet.

## Claims

1. A process for the preparation of 3,4'-dichlorodiphenyl ether from 1,3-dichlorobenzene and 4-chlorophenol, which comprises mixing 1 mol of 4-chlorophenol with 2 to 6 mol of initially introduced 1,3-dichlorobenzene and 1 to 3 mol of potassium carbonate in 1 to 5 mol of a dipolar aprotic solubilizer which boils above 160°C, at temperatures of 160°C to 190°C with stirring, then adding 0.01 to 1 mol-% of basic copper carbonate of the formula Cu(OH)₂·CuCO₃·0.5 H₂O and heating with stirring to 170 to 173°C while removing the water formed by distillation, then optionally adding further 4-chlorophenol with the proviso that from the start the amount of 1,3-dichlorobenzene present in terms of moles is always 1 to 3 mol greater than the totally added amount of 4-chlorophenol and, if desired, additionally adding basic copper carbonate of the formula mentioned if at the start less than 0.1 mol-% had been added, additionally stirring within the last-mentioned temperature range and finally working up after allowing to cool.

2. The process as claimed in claim 1, wherein the reaction is carried out in the presence of 0.1 to 0.9 mol-% of basic copper carbonate.

3. The process as claimed in at least one of claims 1 and 2, wherein the reaction is carried out in the presence of 1 to 2 mol of a dipolar aprotic solubilizer which boils above 160°C.

4. The process as claimed in at least one of claims 1 to 3, wherein the reaction is carried out in N-methylpyrrolidone as the solubilizer.

5. The process as claimed in at least one of claims 1 to 4, wherein the reaction is carried out at temperatures of 170 to 180°C.

6. The process as claimed in at least one of claims 1 to 5, wherein the reaction is carried out with 1 to 1.5 mol of potassium carbonate.

7. The process as claimed in at least one of claims 1 to 6, wherein the reaction is carried out at normal pressure.

8. The process as claimed in at least one of claims 1 to 6, wherein the reaction is carried out at elevated pressure in the presence of a dipolar aprotic solubilizer which boils below 160°C.

9. The process as claimed in at least one of claims 1 to 8, wherein the reaction is carried out batchwise or continuously.

## Revendications

1. Procédé pour la préparation de l'éther 3,4'-dichloro-diphénylique à partir du 1,3-dichlorobenzène et de 4-chlorophénol, caractérisé en ce qu'on mélange 1 mole du 4-chlorophénol avec 2 à 6 moles de 1,3-dichlorobenzène, introduit en premier lieu, et avec 1 à 3 moles de carbonate de potassium dans 1 à 5 moles d'un agent solubilisant aprotique dipolaire, dont le point d'ébullition est supérieur à 160 °C, à des températures de 160 à 190 °C, en agitant, ensuite on ajoute de 0,01 à 1 mole % de carbonate cuivreux alcalin de formule Cu(OH)₂·CuCO₃·0,5 H₂O, puis, en agitant, on chauffe à 170-173 °C, en chassant par distillation l'eau formée, ensuite on ajoute éventuellement encore du 4-chlorophénol à la condition que la quantité de 1,3-dichlorobenzène présente dès le début, soit constamment supérieure, en moles, de 1 à 3 moles à la quantité de 4-chlorophénol ajoutée au total, et éventuellement on ajoute encore du carbonate cuivreux alcalin de formule citée, dans le cas où on aurait ajouté au début moins de 0,1 mole %, on continue à agiter dans le domaine des températures citées en dernier lieu, et ensuite, on complète le traitement après refroidissement.

2. Procédé selon la revendication 1, caractérisé en ce qu'on effectue la réaction en présence de 0,1 à 0,9 mole % de carbonate cuivreux alcalin.

3. Procédé selon au moins l'une des revendications 1 et 2, caractérisé en ce qu'on effectue la réaction en présence de 1 à 2 moles d'un agent de solubilisation aprotique dipolaire, ayant un point d'ébullition supérieur à 160 °C.

4. Procédé selon au moins l'une des revendications 1 et 3, caractérisé en ce qu'on effectue la réaction dans de la N-méthylpyrrolidone en tant qu'agent de solubilisation.

5. Procédé selon au moins l'une des revendications 1 et 4, caractérisé en ce qu'on effectue la réaction à des températures de 170 à 180 °C.

6. Procédé selon au moins l'une des revendications 1 et 5, caractérisé en ce qu'on effectue la réaction avec 1 à 1,5 mole de carbonate de potassium.

7. Procédé selon au moins l'une des revendications 1 et 6, caractérisé en ce qu'on effectue la réaction à la pression normale.

8. Procédé selon au moins l'une des revendications 1 et 6, caractérisé en ce qu'on effectue la réaction sous une surpression, en présence d'un agent de solubilisation aprotique dipolaire, ayant un point d'ébullition inférieur à 160 °C.

9. Procédé selon au moins l'une des revendications 1 et 8, caractérisé en ce qu'on travaille en discontinu ou en continu.
